# EUROPEAN PATENT APPLICATION

(11) **EP 1 475 385 A1**
(43) Date of publication of application: **10.11.2004**
(21) Application number: 03010120.8
(22) Date of filing: 05.05.2003
(51) Int. Cl.: C07H 13/08, A61K 31/7024, A61P 25/00

(54) **Glycoside derivatives of 2-(3,4-dichlorobenzoyl)-cycopropane-1-carboxylic acid**

(71) Applicant: Newron Pharmaceuticals S.p.A., 20091 Bresso (MI) (IT)
(72) Inventor: Benatti, Luca, 20091 Bresso (MI) (IT); Fariello, Ruggero, 20091 Bresso (MI) (IT); Salvati, Patricia, 20091 Bresso (MI) (IT); Pellicciari, Roberto, 20091 Bresso (MI) (IT); Caccia, Carla, 20091 Bresso (MI) (IT)
(74) Representative: Minoja, Fabrizio, Dr.

(57) **Abstract**

The compounds of formula I: 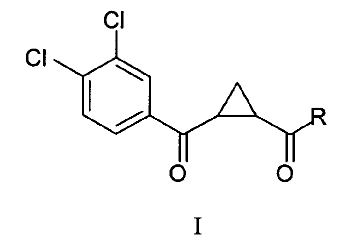 wherein R is a glycoside residue optionally having one or more hydroxy groups alkylated or acylated by C1-C4 alkyl or acyl groups, are long lasting inhibitors of kynurenine 3-monooxygenase (KMO) and potent glutamate (GLU) release inhibitors.

## Description

The present invention refers to novel glycoside derivatives of 2-(3,4-dichlorobenzoyl)-cyclopropane-1-carboxylic acid as long lasting inhibitors of kynurenine 3- monooxygenase (KMO) which are potent glutamate (GLU) release inhibitors.

### BACKGROUND OF THE INVENTION

Metabolites of the kynurenine pathway of tryptophan degradation have been suggested to play an important role in the pathogenesis of several human brain diseases. One of the key metabolites in this pathway, kynurenine, (KYN), is either transaminated to form kynurenate (KYNA), or hydroxylated to the free radical generator 3-OH-KYN. The latter is further degraded to the excitotoxic NMDA receptor agonist QUIN. 3-OH-KYN and QUIN act synergistically, i.e. 3-OH-KYN significantly potentiates the excitotoxic actions of QUIN. The key enzymes in the mammalian brain responsible for the biosynthesis of 3-OH-KYN, (kynurenine 3-monooxygenase, KMO; E.C.1.14.13.9), QUIN (3-hydroxyanthranilate oxygenase) and KYNA (kynurenine aminotransferases (KATs I and II) have been characterized and cloned. KMO is a flavin-containing enzyme localized in outer mitochondria membranes of the liver, placenta, spleen, kidney and brain.

Studies from several laboratories have provided evidence that the shift of KYN pathway metabolism away from the 3-OH-KYN/QUIN branch to increase the formation of the neuroprotectant KYNA in the brain leads to neuroprotection.

Elevations in the brain content of KYNA are of particular interest, since they define KMO as a new molecular target for drug development in the area of neuroprotection. The working mechanism is that inhibition of KMO blocks the synthesis of neurotoxins 3-OH-KYN and QUIN, causes accumulation of KYN upstream the metabolic block, and redirect the metabolism of this latter towards the neuroprotectant KYNA.

Notably, it has been reported that KMO expression increases in inflammatory conditions or after immune stimulation (Saito et al. 1993, *J. Biol. Chem.* 268, 15496.-15503; Chiarugi et al 2001, *Neuroscience* 102; 687-695). 3-OH-KYN, the product of its activity, accumulates in the brain of vitamin B-6 deficient neonatal rats (Guilarte and Wagner, 1987, *J. Neurochem.* 49, 1918-1926) and it causes cytotoxicity when added to neuronal cells in primary cultures (Eastman and Guilarte, 1989, *Brain Res.* 495, 225.-231) or when locally injected into the brain (Nakagami et al. 1996, *Jpn. J. Pharmacol.* 71, 183.-186). Recently, it was reported that relatively low concentrations (nanomolar) of 3-OH-KYN may cause apoptotic cell death of neurons in primary neuronal cultures. Structure-activity studies have in fact shown that 3-OH-KYN, and other o-aminophenols, may be subject to oxidative reactions initiated by their conversion to quinoneimines, a process associated with concomitant production of oxygen derived free radicals (Hiraku et al. 1995 *Carcinogenesis* 16, 349-356). The involvement of these reactive species in the pathogenesis of ischemic neuronal death has been widely studied in the last several years and it has been shown that oxygen derived free radicals and glutamate mediated neurotransmission co-operate in the development of ischemic neuronal death (Pellegrini-Giampietro et al. 1990, *J. Neurosci.* 10, 1035-1041).

It was also recently demonstrated that KMO activity is particularly elevated in the iris-ciliary body and that neo-formed 3-OH-KYN is secreted into the fluid of the lens. An excessive accumulation of 3-OH-KYN in the lens may cause cataracts and KMO inhibitors may prevent this accumulation (Chiarugi et al. 1999; *FEBS Letters,* 453; 197-200).

As already mentioned, KMO activity is required for tryptophan catabolism and synthesis of quinolinic acid (QUIN). QUIN is an agonist of a subgroup of NMDA receptors (Stone and Perkins, 1981 *Eur. J. Pharmacol.* 72, 411-412) and when directly injected into brain areas it destroys most neuronal cell bodies sparing fibers *en passant* and neuronal terminals (Schwarcz et al. 1983 *Science* 219, 316-318). QUIN is a relatively poor agonist of the NMDA receptor complex containing either NR2C or NR2D subunits, while it interacts with relatively high affinity with the NMDA receptor complex containing NR2B subunits (Brown et al. 1998, *J. Neurochem.* 71, 1464-1470). The neurotoxicity profile found after intrastriatal injection of QUIN closely resembles that found in the basal nuclei of Huntington's disease patients: while most of the intrinsic striatal neurons are destroyed, NADH-diaphorase-staining neurons (which are now considered able to express nitric oxide synthetase) and neurons containing neuropeptide Y seem to be spared together with axon terminals and fiber *en passant* (Beal et al. 1986 *Nature* 321, 168-171).

*In vitro,* the neurotoxic effects of the compound have been studied in different model systems with variable results: chronic exposure of organotypic cortico-striatal cultures to submicromolar concentration of QUIN causes histological signs of pathology (Whetsell and Schwarcz, 1989, *Neurosci. Lett.* 97, 271-275), similar results have been obtained after chronic exposure of cultured neuronal cells (Chiarugi et al 2001, *J*. *Neurochem.* 77, 1310-1318).

In models of inflammatory neurological disorders such as experimental allergic encephalitis (Flanagan et al. 1995, *J. Neurochem.* 64, 1192-1196), bacterial and viral infections (Heyes et al. 1992 *Brain* 115, 1249-1273; Espey et al. 1996, *AIDS* 10, 151-158), forebrain global ischemia or spinal trauma, brain QUIN levels are extremely elevated (Heyes and Nowak, 1990 *J. Cereb. Blood Flow Metab.* 10, 660-667; Blight et al. 1995 *Brain* 118, 735-752). This increased brain QUIN concentration could be due to either an elevated circulating concentration of the excitotoxin or to an increased de novo synthesis in activated microglia or in infiltrating macrophages. In retrovirus-infected macaques, it has been proposed that most of the increased content of brain QUIN (approximately 98%) is due to local production. In fact, a robust increase in the activities of IDO, KMO and kynureninase has been found in areas of brain inflammation (Heyes et al. 1998; *FASEB J.* 12, 881-896).

Previous studies have shown that agents able to increase brain KYNA content cause sedation, mild analgesia, increase in the convulsive threshold and neuroprotection against excitotoxic or ischemic damage (Carpenedo et al 1994 *Neuroscience* 61, 237-244; Moroni et al. 1999 *Eur. J. Pharmacol.* 375, 87-100; Cozzi et al. 1999; *J, Cereb. Blood Flow & Metab.* 19, 771-777).

In addition to the above reported evidences, it has been recently demonstrated that a number of compounds able to increase brain KYNA formation may cause a robust decrease in glutamate (GLU) mediated neurotransmission by reducing GLU concentrations in brain extracellular spaces (Carpenedo et al 2001 , *Eur. J. Neuroscience* 13, 2141-2147).

Compounds endowed with KMO inhibiting activity may therefore be used for the treatment of a number of degenerative or inflammatory conditions in which an increased synthesis in the brain of QUIN, 3-OH-KYN are involved and may cause neuronal cell damage. These compounds in fact by inhibiting the KMO enzyme prevent the synthesis of both 3-OH-KYN and QUIN, and concomitantly cause KYNA to increase in the brain.

2-substituted benzoyl-cycloalkyl-1-carboxylic acid derivatives having KMO inhibiting activity are disclosed in WO 98/40344. In particular one of said compounds, 2-(3,4-dichlorobenzoyl)-cyclopropane-1-carboxylic acid, was reported to have an interesting activity, with an IC₅₀ for KMO inhibition of 0.18 µM, but its potency and pharmacokinetics properties were less than satisfactory.

### DESCRIPTION OF THE INVENTION

It has now been found that certain glycoside derivatives of 2-(3,4-dichlorobenzoyl)-cyclopropane-1-carboxylic acid have favourable and long lasting inhibitory activities on both KMO and on GLU release.

The present invention accordingly provides compounds of formula I 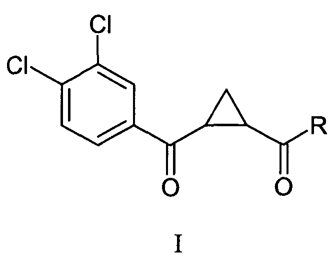 wherein R is a glycoside residue optionally having one or more hydroxy groups alkylated or acylated by C1-C4 alkyl or acyl groups.

By the term glycoside residue, a mono-, di- or oligosaccharide is meant.

R is preferably an optionally alkylated or acylated beta -D-glucopyranosyloxy or 6-deoxygalactopyranosyloxy residue.

The galactopyranosyl residue is particularly preferred.

The compounds of the invention have asymmetric carbon atoms so that they exist either as racemic mixtures or as individual optical isomers (enantiomers). Moreover the compounds of the invention can also be E- or Z- isomers or E-, Z- mixtures thereof. The present invention includes within its scope all the possible isomers and their mixtures.

The isomers E- of the compounds of formula I are preferred.

Preferably, the carbon atoms in positions 1 and 2 of the cyclopropane ring have the S configuration.

α-d-galactopyranosyl (1*s*, 2*s*)-2-(3,4-dichlorobenzoyl)-cyclopropane-1-carboxylate, hereinafter referred to asNW-1100, is particularly preferred.

The invention also concerns pharmaceutical compositions comprising a compound of formula I as the active ingredient as well as the use of said compounds I for the preparation of medicaments for use as kynurenine-3-hydroxylase inhibitors.

The compounds of the invention may be obtained by a process comprising the reaction of 2-(3,4-dichlorobenzoyl)-cyclopropane-1-carboxylic acid with a suitably protected saccharide derivative, optionally followed by the removal of the protective groups present on the saccharide hydroxy groups.

Examples of suitable saccharide derivatives include 1,2,3,4-di-O-isopropylidene-galactopyranose, 1,2,3,4-di-O-isopropylidene-glucopyranose, glucopyranosyl bromide tetraacetate or tetrabenzoate, glucopyranosyl chloride tetraacetate or tetrabenzoate, galactopyranosyl bromide tetraacetate or tetrabenzoate, galactopyranosyl chloride tetraacetate or tetrabenzoate and the like. Preferably, 2-(3,4-dichlorobenzoyl)-cyclopropane-1-carboxylic acid is reacted with 1,2,3,4-di-O-isopropylidene-galactopyranose or glucopyranose in the presence of a condensing agent such as carbonyldiimidazole, dicyclohexylcarbodiimide or the like, in anhydrous solvents and under inert atmosphere. The obtained compounds may then be transformed into the desired compounds of formula I by treatment with organic acids, e.g. with trifluoroacetic or trichloroacetic acid in halogenated hydrocarbons, ethers, aliphatic or aromatic hydrocarbons, etc.

The starting material 2-(3,4-dichlorobenzoyl)-cyclopropane-1-carboxylic acid may be prepared as disclosed in WO 98/40344, which is herein incorporated by reference.

2-(3,4-dichlorobenzoyl)-cyclopropane-1-carboxylic acid may be used either in form of an isomer or enantiomer mixture or as a single isomer and/or enantiomer.

The isomer and/or enantiomer separation, if desired, may be carried out according to known methods either before or after the reaction with the saccharide derivative. It is however preferred to react a pure isomer and/or enantiomer of 2-(3,4-dichlorobenzoyl)-cyclopropane-1-carboxylic acid which may be obtained by conventional isomer/enantiomer separation methods or by stereospecific synthesis.

An example of a stereospecific synthesis starting from 1-menthol and succinic anhydride will be disclosed in the Examples.

The compounds of the invention are potent KMO inhibitors and they can modify the formation of all the neuroactive compounds formed along the pathway. In particular, they inhibit the formation of 3-OH-KYN and its metabolites in the pathway leading to QUIN. More particularly, the compounds of this invention are able to increase brain KYNA content and to decrease excitatory glutamatergic neurotransmission with a long-lasting and particularly favorable time course.

The compounds of the invention may therefore be used for the treatment of a number of degenerative or inflammatory conditions in which an increased synthesis in the brain of QUIN, 3-OH-KYN or increased release of GLU are involved and may cause neuronal damage. Examples of said conditions include:
1) Neurodegenerative disorders including Parkinson's syndrome, Huntington's chorea, Senile Dementia Alzheimer's type, Amiotrophic Lateral Sclerosis;
2) Inflammatory disorders of the central and/or peripheral nervous system including multiple sclerosis (see: Chiarugi et al. *Neuroscience* 2001, 102, 687-695; Chiarugi et al. *J. Leukoc. Biol.* 2000, 68, 260-266), Guillain Barrè Syndrome and other neurophaties
3) Infectious disease caused by viral (including AIDS see:Heyes et al. *Annals Neurol.* 1991, 29, 202-209), bacteria and other parasites including malaria, septic shock, etc.
4) Immunitary disorders and therapeutic treatment aimed at modifying biological responses (for instance administrations of interferons or interleukins, see: Brown et al. *Cancer Res.* 1989, 49, 4941-4945).
5) Neoplastic disorders including lymphomas and other malignant blood disorders.
6) Convulsive Disorders, including variants of *Grand mal* and *petit mal* epilepsy and Partial Complex epilepsy (see: Carpenedo et al. 1994, *Neuroscience* 61, 237-244).
7) Ischemic disorders including stroke (focal ischemia); cardiac arrest or insufficiency and hemorragic shock (global brain ischemia), carbon monoxide poisoning, near drawning (see: Cozzi et al. 1999, *J. Cereb. Blood Flow Metab.* 19, 771-777).
8) Traumatic damage to the brain and spinal cord.
9) Tremor syndromes and different movement disorders (diskynesia).
10) Psychiatric disorders including anxiety, insomnia, depression and schizophrenia.
11) Nicotine addiction (kynurenate is an antagonist of nicotinic receptors). Other addictive disorders including alcoholism, cannabis, benzodiazepine, barbiturate, morphine and cocaine dependence (see: Albuquerque et al. 2001, *J*. *Neurosci.* 21, 7463-7473).
12) Cataract formation and aging of the eye (see: Chiarugi et al. 1999; *FEBS Letters* 453, 197-200).

For the considered therapeutic uses, the compounds of the invention will be administered to the affected patients in form of pharmaceutical compositions suitable for the oral, parenteral, transmucosal or topical administration.

The pharmaceutical compositions may be prepared following conventional methods.

For example, the solid oral forms may contain, together with the active compound, diluents, e.g. lactose, dextrose, saccharose, sucrose, cellulose, corn starch or potato starch; lubricants, e.g. silica, talc, stearic acid, magnesium or calcium stearate, and/or polyethylene glycols; binding agents, e.g. starches, arabic gum, gelatin, methylcellulose, carboxymethylcellulose or polyvinyl pyrrolidone; disaggregating agents, e.g. a starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. Said pharmaceutical preparations may be manufactured in known manner, for example, by means of mixing, granulating, tabletting, sugar-coating, or film-coating processes.

The liquid dispersions for oral administration may be e.g. syrups, emulsions and suspensions.

The syrups may contain as carrier, for example, saccharose or saccharose with glycerine and/or mannitol and/or sorbitol.

The suspensions and the emulsions may contain as carrier, for example, a natural gum, agar, sodium alginate, pectin, methylcellulose, carboxymethylcellulose, or polyvinyl alcohol.

The suspension or solutions for intramuscular injections may contain a pharmaceutically acceptable carrier, e.g. sterile water, olive oil, ethyl oleate, glycols and optionally local anaesthetics. The solutions for intravenous injections or infusions may contain as carrier, for example, sterile water, isotonic saline solutions or propylene glycol.

The suppositories may contain a pharmaceutically acceptable carrier, e.g. cocoa butter, polyethylene glycol, a polyoxyethylene sorbitan fatty acid ester surfactant or lecithin.

For the use in ophthalmology, the compounds may be formulated as eye-drops in a sterile carrier, usually an isotonic saline solution.

The dosage level will depend on the age, weight, conditions of the patient and on the administration route even though it will typically range from about 10 to about 1000 mg pro dose, from 1 to 5 times daily.

The following examples illustrate the invention in more detail.

### Example 1

### Synthesis of α-d-galactopyranosyl (1s, 2s)-2-(3,4-dichlorobenzoyl)-cyclopropane-1-carboxylate (NW-1100)

### a) (-)-Dimenthyl Succinate

To a solution of succinic anhydride (15.2 g, 0.152 mol) and *l*-menthol (47.5 g, 0.304 mol) in dry toluene (120 ml), under magnetic stirring, was added *p*-toluensulfonic acid (0.190 g, 1.04 10⁻³ mol). The mixture was heated under reflux for 24 h and the theoretical amount of water (2.73 ml) was collected. The cooled mixture was diluted with petroleum ether (200 ml) and poured into a 2.5:1:2 mixture of saturated aqueous sodium bicarbonate solution, methanol and water (550 ml). The organic phase was separated and the aqueous layer extracted with petroleum ether (3x100 ml). The collected organic layers were washed with saturated sodium chloride (1x100 ml) and dried over sodium sulphate. The solvent was removed with a rotary evaporator and the resulting crude product recrystallized with methanol. 54 g of pure (-)-dimenthyl succinate (3) were obtained (89%).
mp: 61-62°C
[α]_{D}²⁵ = -87 (c = 1, CHCl₃)

### b) (+)-Dimenthyl (1S, 2S)-Cyclopropane-1,2-dicarboxylate.

To 180 ml of dry tetrahydrofuran (THF), cooled to -20°C, a 2.5 M solution of butyllithium in hexane (56.9 ml, 142.2 mmol) was added. 2,2,6,6-tetramethylpiperidine (24 ml, 142.2 mmol) was added dropwise over a period of 10 minutes. The resulting solution of lithium 2,2,6,6-tetramethylpiperidide (LTMP) was cooled to -78°C and stirred for 30 minutes. A solution (-)-dimenthyl succinate (26.75 g, 67.7 mmol) in THF (60 ml) was then added over a period of 1 h. The resulting yellow solution was stirred for 1 h. Then, bromochloromethane (4.39 ml, 67.7 mmol) was added and the reaction mixture stirred for 2 h. The reaction was quenched by adding isobutyraldehyde (22.46 ml, 27.08 mmol). After stirring for additional 30 minutes, the mixture was poured into ice-cooled 1N hydrochloric acid (250 ml) and the aqueous layer was extracted with diethyl ether (3x150 ml). The combined organic layers were washed with saturated sodium chloride (250 ml), dried over sodium sulphate and concentrated with a rotary evaporator. The residue was chromatographed on silica gel (petroleum ether/diethyl ether = 98/2). An additional flash chromatography on silica gel (petroleum ether/diethyl ether = 98/2) afforded of the pure title product in 33% yield.
mp: 95-96°C
[α]_{D}²⁵ = -18.8 (c = 1, CHCl₃)
¹H-NMR (CDCl₃) δ: 0.70-2.20-(complex, 20 H); 0.75 (d, 6H, J = 7 Hz); 0.9 (d, 9H, J = 6.8 Hz); 2.15 (dd, 2H, J = 7.6, 8.7 Hz); 4.7 (dt, 2H, J = 4.3, 10.7 Hz).

¹³C-NMR (CDCl₃) δ: 15.2; 16.4; 20.6; 21.9; 22.2; 23.6; 26.3; 31.3; 34.2; 40.8; 47.0; 74.9; 171.2.

### c) (+)-(1S, 2S)-Cyclopropane-1,2-dicarboxylic acid.

To a solution of (+)-dimenthyl (1*S*, 2*S*)-cyclopropane-1,2-dicarboxylate (8.8 g, 21.62 mmol) in methanol (38 ml), an aqueous solution (5 ml) of potassium hydroxide (4.32 g; mmol) was added. The mixture was heated to 60°C for 4 h then cooled to room temperature. The reaction mixture was diluted with water (40 ml) and extracted with diethyl ether (4x40 ml). The aqueous layer was acidified with 3N hydrochloric acid, saturated with sodium chloride and extracted with diethyl ether (6x40 ml). The combined organic layers were dried over sodium sulphate and concentrated with a rotary evaporator. 2.27 g of the title product were obtained after sublimation (80% yield).
mp: 168-169°C
[α]_{D}²⁰ = +224.9 (c = 1, EtOH)
¹H-NMR (CDCl₃+CD₃OD) δ: 1.45 (t, 2H, J = 8.2 Hz); 2.1 (t, 2H, J = 7 Hz); 7.9 (br, 2H).

### d) (+)-Dimethyl (1S, 2S)-Cyclopropane-1,2-dicarboxylate.

A solution of (+)-(1S, 2*S*)-cyclopropane-1,2-dicarboxylic acid (2.2 g, 16.9 mmol) in oxalyl chloride (35 ml) was stirred under argon at room temperature for 4 h.. Oxalyl chloride was then removed with a rotary evaporator and the oily residue dissolved in dry methanol (100 ml). Stirring was continued for 12 h and methanol evaporated. The residue was chromatographed on silica gel (petroleum ether/ethyl acetate = 85/15-7/3) thus affording 2.48 g of (+)-dimethyl (1*S*, 2*S*)-cyclopropane-1,2-dicarboxylate (93% yield).
[α]_{D}²⁴ = +218 (c = 5, CH₂Cl₂)
¹H-NMR (CDCl₃) δ: 1.45 (t, 2H, J = 8.2 Hz); 2.1 (t, 2H, J = 7 Hz); 3.65 (s, 6H).
¹³C-NMR (CDCl₃) δ: 15.0; 21.9; 51.8; 171.9.

### e) (+)-(1S, 2S)-Cyclopropane-1,2-dicarboxylic Acid Monomethyl Ester.

To a solution of (+)-dimethyl (1*S*, 2*S*)-cyclopropane-1,2-dicarboxylate (3.68 g, 23.25 mmol) in methanol (23 ml), a methanolic solution (13 ml) of potassium hydroxide (1.44 g; 25.72 mmol) was added. The mixture was heated at 60°C for 5h then cooled and poured into water (30 ml). The aqueous layer was extracted with ethyl acetate (1x10 ml), acidified with 3N hydrochloric acid and extracted again with ethyl acetate (4x10 ml). The combined organic layers were washed with saturated sodium chloride (1x10 ml), dried over sodium sulphate and concentrated under vacuum. 2.69 g of the title product were obtained (80% yield).
[α]_{D}²⁴ = +245 (c = 1, CH₂Cl₂)
¹H-NMR (CDCl₃) δ: 1.45 (m, 2H); 2.15 (m, 2H, J = 7 Hz); 3.65 (s, 3H); 10.7 (br, 1H).

### f) (+)-Methyl (1S, 2S)-2-(3,4-Dichlorobenzoyl)-cyclopropane-1-carboxylate.

A solution of (+)-(1*S*, 2*S*)-cyclopropane-1,2-dicarboxylic acid monomethyl ester (2.64 g, 18.34 mmol) in oxalyl chloride (100 ml) was stirred at room temperature under argon for 20 h. Oxalyl chloride was then removed with a rotary evaporator and the residue dissolved in 1,2-dichlorobenzene (50 ml). To the resulting solution, kept at 0°C, aluminium trichloride (7.33 g, 55.01 mmol) was added portionwise during 30 minutes. The mixture was heated at 60°C for 3h, then cooled and poured into ice/3N hydrochloric acid. The aqueous layer was extracted with ethyl acetate (4x15 ml) and the combined organic layers washed with saturated sodium chloride, dried over sodium sulphate and concentrated under vacuum. Excess 1,2-dichlorobenzene was eliminated by distillation with a membrane pump. The residue was purified by flash chromatography on silica gel (petroleum ether/ethyl acetate = 85/15) affording 3.45 g of title product (69% yield).
[α]_{D}²⁰ = +168 (c = 1.09, CH₂Cl₂)
¹H-NMR (CDCl₃) δ: 1.65 (t, 2H, J = 7.2 Hz); 2.45 (m, 1H, J = 6.8, 3.9 Hz); 3.10 (m, 1H, J = 7.2, 3.8 Hz); 3.75 (s, 3H); 7.55 (d, 1H, J = 8.3 Hz); 7.85 (dd, 1H, J = 8.3, 2 Hz); 8.10 (d, 1H, J = 2.1 Hz).

### g) (+)-(1S, 2S)-2-(3,4-Dichlorobenzoyl)-cyclopropane-1-carboxylic acid.

To a solution of (+)-methyl (1S, 2S)-2-(3,4-dichlorobenzoyl)-cyclopropane-1-carboxylate (3.42 g, 12.54 mmol) in dioxane (100 ml), an aqueous solution (34 ml) of potassium hydroxide (0.704 g, 12.54 mmol) was added. The resulting mixture was stirred at room temperature for 2 h then diluted with water (80 ml). The aqueous layer was extracted with ethyl acetate (1x40 ml), acidified with 3N hydrochloric acid and extracted with ethyl acetate (4x40 ml). The combined organic layers were washed with saturated sodium chloride, dried over sodium sulphate and concentrated with a rotary evaporator. The residue was purified by flash chromatography on silica gel (petroleum ether/ethyl acetate = 6/4) affording 3.10 g of the title product (95% yield).
[α]_{D}²² = +142 (c = 0.98, acetone)
¹H-NMR (CDCl₃+CD₃OD) δ: 1.65 (m, 2H); 2.45 (m, 1H); 3.10 (m, 1H); 3.75 (s, 3H); 7.55 (d, 1H, J = 8.2 Hz); 7.85 (dd, 1H, J = 8.2, 2.1 Hz); 8.15 (d, 1H, J = 2.1 Hz); 10.0 (br, 1H).
¹³C-NMR (CDCl₃+CD₃OD) δ: 18.12; 24.98; 25.93; 127.84; 130.96; 131.67; 133.67; 137.37; 173.36; 195.54.

### h) 1,2;3,4-(Di-O-isopropylidene)-α-D-galactopyranosyl (1S, 2S)-2- (3,4-Dichlorobenzoyl)-cyclopropane-1-carboxylate.

To a solution of (+)-(1*S*, 2*S*)-2-(3,4-dichlorobenzoyl)-cyclopropane-1-carboxylic acid (0.505 g, 1.95 mmol) in methylene chloride (9 ml) carbonyldiimidazole, CDI, (0,348 g, 2.14 mmol) was added and the reaction mixture left under stirring for 2 h at room temperature under an argon atmosphere. Then, a solution of 1,2;3,4-(di-O-isopropylidene)-α-D-galactopyranose (0.508 g, 1.95 mmol) in methylene chloride (9 ml) was added. After stirring 16 h at room temperature, the reaction was concentrated with a rotary evaporator. The residue was purified by flash chromatography on silica gel (petroleum ether/ethyl acetate = 8/2) affording 0.846 g of title compound (87% yield).
¹H-NMR (CDCl₃) δ: 1.28 (s, 3H); 1.30 (s, 3H); 1.42 (s, 3H); 1.43 (s, 3H); 1.59-1.64 (dd, 2H, J = 6.6, 7.6 Hz); 2.36-2.45 (m, 1H, J = 3.8, 6.6, 8.0 Hz); 3.04-3.13 (m, 1H, J = 3.8, 6.6, 7.6 Hz); 3.97-4.03 (m, 1H, J = 1.6, 4.2, 7.4 Hz); 4.15-4.36 (complex, 4H); 4.56-4.62 (dd, 1H, J = 2.4, 7.8 Hz); 5.50-5.53 (d, 1H, J = 5 Hz); 7.51-7.55 (d, 1H, J = 8.4 Hz); 7.79-7.84 (dd, 1H, J = 2.0, 8.2 Hz); 8.05-8.06 (d, 1H, J = 2.0 Hz).

### i) α-D-Galactopyranosyl (1S, 2S)-2-(3,4-Dichlorobenzoyl)-cyclopropane-1-carboxylate (NW-1100).

To a solution of 1,2;3,4-(di-O-isopropylidene)-α-D-galactopyranosyl (1*S*, 2*S*)-2-(3,4-dichlorobenzoyl)-cyclopropane-1-carboxylate (3.737 g, 7.467 mmol) in methylene chloride (150 ml) trifluoroacetic acid (75 ml) was added. The reaction mixture was left under stirring for 24 h at room temperature, then concentrated under vacuum. The residue was taken up with diethyl ether and concentrated with a rotary evaporator several times. Then, the white solid obtained was minced in hexane, filtered and dried with a high vacuum pump affording 2.90 g of NW-1100(92% yield).
¹H-NMR (CD₃OD δ: 1.48-1.67 (m, 2H); 2.25-2.37 (m, 1H); 3.16-3.25 (m, 1H); 3.43-4.37 (complex, 6H); 5.08-5.10 (d, 1H, J = 3.2 Hz); 7.65-7.69 (d, 1H, J = 8.4 Hz); 7.92-7.97 (dd, 1H, J = 8.4, 2.0 Hz); 8.11-8.12 (d, 1H, J = 2.0 Hz).
¹³C-NMR (CD₃OD) δ: 17.01; 24.17; 25.30; 64.53; 68.86; 69.68; 72.41; 127.61; 129.77; 130.79; 133.84; 136.68; 137.39; 171.68; 195.00.

### Example 2

### In vitro enzyme assay

Rat brain mitochondria preparation: Male Wistar rats were sacrificed and brain quickly removed, cortex was dissected out on ice and homogenized in 2.5 volumes of ice-cold 0.32 M saccarose using a glass-Teflon homogenizer. After centrifugation at 600 g for 10 min at 4°C, the supernatant was stored and the pellet was washed and centrifuged again. The two supernatants were pooled and centrifuged at 10,000 g for 10 min at 4°C. The pellet was washed in fresh saccarose and centrifuged twice at 15,000 g for 10 min at 4°C. The pellet (corresponding to the mitochondrial fraction) was resuspended in 2.5 volumes of ice-cold 20 mM potassium phosphate buffer containing 0.14 M KCl, pH 7.0. Protein content was determined by BCA protein assay kit from Pierce.

In vitro KMO activity determination: The enzyme activity was evaluated by measuring by HPLC the formation of 3-OH-KYN in homogenates incubated in the presence of a fixed concentration of KYN (close to the enzyme Kₘ that in our experimental conditions is 30 µM)

The reaction mixture contained 50 µl of homogenate, 50 µl of 2 mM MgCl₂, 0.4 mM NADPH in 50 mM potassium phosphate buffer, pH 7.5, 50 µl of KYN (50µM) and 50 µl of buffer or different concentration of the test compound with or without 30 minutes of pre-incubation. The incubation was carried out for 60 min at 37°C and stopped by adding 200 µl of 1 M HClO₄. Samples were centrifuged at 12,000 rpm for 10 min at 4°C. The supernatant (30 µl) was directly injected onto the HPLC with electrochemical detector (Coulochem II, ESA) at working potential set at + 0.5 V. Isocratic separation was achieved using a reverse phase C18 (25 cm, 5 µ size) Alltech column, with 0.05 M phosphate buffer (pH 3.2) containing 1 mM octansulphonate, 0.1 mM EDTA and 6% acetonitrile, flowing at 1 ml/min.

IC₅₀ values were determined from three independent inhibition curves using at least seven different inhibitor concentrations using non linear regression analysis (best fitting aided-computer program.

Results: The compound of Example 1,NW-1100 inhibited *in vitro* brain KMO in the nMolar range (IC₅₀= 66 nM). When the compound was preincubated with mitochondria preparation for 30 min before adding the substrate (KYN), the IC₅₀ was not significantly different. This result indicates that the KMO inhibition is due to NW-1100 and is not affected by metabolising enzymes present in the mitochondria preparation.

Other compounds of the invention proved to be active in the same range of concentration.

### Example 3

### In vivo experiments

Measurement of hippocampal kyna levels: Microdialysis was performed in adult, male rats (Charles-River; 200-250 g). Under anesthesia, a microdialysis probe (CMA/10, membrane length: 2 mm, Carnegie Medicin, Stockholm, Sweden) was implanted vertically into the brain, extending throughout the dorsal hippocampus. On the next day, the probe was connected to a microperfusion pump, and the hippocampus of the unanesthetized rat was perfused with Ringer solution at a speed of 1 microliter/min. Dialysate was collected every 30 min.

The concentration of KYNA in the dialysis samples was determined by HPLC with fluorimetric detection, using the analytical procedure described by Wu et al. (Eur. J. Neurosci., 4: 1264-1270,1992).

Striatal GLU release: Transcerebral microdialysis tubing having an exposed length of 1 mm for each caudate, were implanted through small burr holes drilled in the skull of anesthetized male Wistar rats. Approximately twelve hours after surgery, in freely moving animals, the membranes were perfused at a flow rate of 3.5 µl/min. After a washout period of approximately one hour, fractions were collected to determine the basal output of KYNA and of GLU.

KYNA was measured using HPLC with post column derivatization and fluorometric detection as previously reported. HPLC separation and fluorometric detection was also used for quantitative measurements of GLU (Carpenedo et al 2001, Europ. J. Neuroscience 13, 2141-2147).

Results: KYNA extracellular concentration, in the hippocampus, was 2.6 ± 0.2 nM (mean ± SEM) under basal conditions, and no significant changes in these concentrations were found by perfusing the animals for at least 24 h. Systemic administration of the compounds of the invention, particularly of NW-1100(25 and 50 mg/kg i.p.),dose-dependently increased brain KYNA content and the maximum increase (26.7± 2.2 nM, P< 0.01, with 50 mg/kg ip) was reached 6 hours after drug administration. A significant increase (P<0.05) in dialysate KYNA concentration was still present up to 24 h after NW-1100 with both 25 and 50 mg/kg i.p.

KYNA extracellular concentration, in the head of the caudate nuclei, was 13.2 ± 0.1 nM. GLU concentration in the same brain nuclei was 4.3 ± 0.9 µM (n = 90 from 30 rats) and no significant changes in these concentrations were found by perfusing the animals for at least 24 h. Systemic administration of NW-1100 (30-100 µmol/kg i.p.) dose-dependently caused a dramatic decrease (up to -78 ± 8% in 2 hrs with the highest dose) in GLU concentrations. The decrease was long lasting as it reached a maximum two hrs after NW-1100 administration, but was still present (-65%) 24 h later. In the same biological fluid an highly significant increased brain KYNA content was observed; the maximum increase (+750%) was reached 180 min after drug administration. A significant increase (+280%) in dialysate KYNA concentration was still present up to 24 h after NW-1100 (100 µmol/kg i.p.).

From the reported data, it turns out that the compounds of the invention, and in particular NW-1100, are endowed with potent *in vitro* inhibitory effect on the enzyme KMO superior to that of other inhibitors described in the literature, particularly in comparison with the compounds disclosed in WO 98/40344. *In vivo,* after systemic administration, it induces significant and long lasting increase in KYNA levels in different brain areas while contemporaneously suppressing GLU release in the caudate. This effect closely parallel the increase in KYNA extracellular levels observed in the same area.

These results demonstrate that NW-1100is a potent and long lasting KMO inhibitor provided with an interesting pharmacokinetic profile. Its action both *in vitro* and *in vivo* seems significantly improved when compared with that of published compounds because of the long duration of its effects. This long duration might be particularly useful to avoid rebound side effects that are possible when the inhibitory effects on the enzyme rapidly fade. The most relevant finding is, however, the fact that NW-1100potently inhibits GLU release in the brain and this effect is still present 24 hours after a single systemic administration.

## Claims

1. Compounds of formula I : 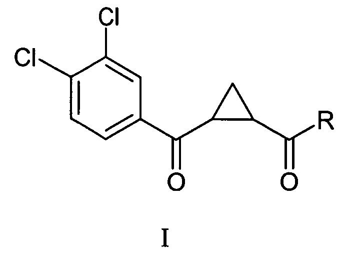 wherein R is a glycoside residue optionally having one or more hydroxy groups alkylated or acylated by C1-C4 alkyl or acyl groups.

2. Compounds according to claim 1 wherein R is the residue of a mono-, di- or oligosaccharide.

3. Compounds according to claim 1 or 2 wherein R is an optionally alkylated or acylated beta -D-glucopyranosyloxy or 6-deoxygalactopyranosyloxy residue.

4. A compound according to claim 3 wherein R is the galactopyranosyl residue.

5. A compound according to any one of claims 1-4 wherein the carbon atoms in positions 1 and 2 of the cyclopropane ring have the S configuration.

6. α-d-galactopyranosyl (1*S*, 2*S*)-2-(3,4-dichlorobenzoyl)-cyclopropane-1-carboxylate.

7. Pharmaceutical compositions comprising a compound of formula I of claims 1-6 as the active ingredient, in admixture with a suitable carrier.

8. The use of compounds of claims 1-6 for the preparation of medicaments for use as KMO inhibitors.
